(19) Europäisches Patentamt
European Patent Office
Office européen des brevets

(11) EP 0 748 204 B1

(12) EUROPEAN PATENT SPECIFICATION

(45) Date of publication and mention of the grant of the patent:
**25.11.1998 Bulletin 1998/48**

(21) Application number: **95911115.4**

(22) Date of filing: **27.02.1995**

(51) Int. Cl.$^6$: **A61K 7/06**

(86) International application number:
**PCT/US95/02383**

(87) International publication number:
**WO 95/23581 (08.09.1995 Gazette 1995/38)**

# (54) HAIR CARE COMPOSITIONS PROVIDING HAIR CONDITIONING AND STYLE RETENTION

HAARFIXIERUNG- UND KONDITIONIERUNGSPFLEGEMITTEL

PRODUITS CAPILLAIRES A EFFETS CONDITIONNEUR ET COIFFANT

(84) Designated Contracting States:
**AT BE CH DE DK ES FR GB GR IE IT LI LU NL PT SE**

(30) Priority: **01.03.1994 US 204120**

(43) Date of publication of application:
**18.12.1996 Bulletin 1996/51**

(73) Proprietor:
**THE PROCTER & GAMBLE COMPANY**
**Cincinnati, Ohio 45202 (US)**

(72) Inventor:
**WERENSKI, Stephen, Paul**
**Cincinnati, OH 45242-6303 (US)**

(74) Representative:
**Woof, Victoria et al**
**Patent Dept.,**
**Procter & Gamble Technical Centres Limited,**
**Rusham Park,**
**Whitehall Lane**
**Egham TW20 9NW (GB)**

(56) References cited:
**EP-A- 0 468 721**

- **CHEMICAL ABSTRACTS, vol. 119, no. 4, 26 July 1993, Columbus, Ohio, US; abstract no. 34088, A. FUKINISHI 'Shampoos containing high molecular weight silicones' & JP,A,5 058 852 (SANYO CHEMICAL IND LTD.) 9 March 1993**
- **PATENT ABSTRACTS OF JAPAN vol. 13, no. 510 (C-654) (3858) 15 November 1989 & JP,A,01 203 314 (SHISEIDO CO. LTD.)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.6/3.4

## Description

### TECHNICAL FIELD

The present invention relates to hair care compositions comprising a silicone conditioning agent, an emulsifying agent, a cationic cosmetic agent, a hair setting agent, and an aqueous carrier.

### BACKGROUND OF THE INVENTION

The desire to have hair retain a particular shape is widely held. Two common methodologies of accomplishing this are permanent chemical alteration of the hair and temporary alteration. A temporary alteration is one which can be removed by water or by shampooing. Temporary alteration is commonly accomplished by means of the application of a hair care composition to dampened hair after shampooing and/or conditioning and prior to drying and/or styling. The materials used to provide hair styling benefits in these compositions have generally been film forming or adhesive resins and have been typically applied in the form of mousses, tonics, lotions, hairsprays, and gels. In typical hair care compositions, hair styling is achieved by the use of resins, such as AMPHOMER, supplied by National Starch Chemical Co., and GANTREZ SP 225R, supplied by GAF Corp. When such hair styling resins are incorporated into hair care compositions, to provide style hold, the tactile feel of the hair becomes stiff and hence less desirable. It is therefore, desirable to also add a conditioner to impart softer hair feel upon drying of the styling resin. Silicone materials have been taught for use in hair care compositions and can be highly effective at conditioning the hair. Especially preferred for imparting a soft hair feel are high molecular weight silicones, commonly referred to as silicone gums. However, these silicone gums are extremely difficult to formulate into water or ethanol based hair care compositions. This is because the silicone gum is too viscous to incorporate or disperse into typical hair care compositions. The high viscosity of the silicone gum leads to difficulties in handling and mixing in the manufacturing process.

One conventional way to reduce the viscosity of silicone gums is by adding a volatile solvent, such as cyclomethicone. The use of volatile solvents reduces the viscosity of the silicone gum to a point where it can be easily incorporated into an emulsion before addition to the hair care composition. However, the volatile solvents can result in negative aesthetic and hair feel properties. Cyclomethicone, for example, can cause the hair to appear and feel oily and less desirable. It may also be desirable to limit the use of the volatile solvents to ensure compliance with respect to various regulatory laws limiting the use or amount of such materials.

Another, method to reduce the viscosity of the silicone gum is to add a non-volatile solvent, such as a silicone fluid to the silicone gum. Silicone fluids, such as polydimethylsiloxane, are known to reduce the viscosity of the high molecular weight silicone gum without incurring the negative hair appearance and feel properties associated with volatile solvents.

Typically, silicone gum as well as mixtures of silicone gum and fluid are emulsified using an anionic surfactant as the emulsifying agent. Although the use of anionic surfactants is desirable in shampoo compositions, it is not desirable to use anionic surfactants in hair care compositions containing cationic cosmetic agents, such as mousses, tonics, lotions, hairsprays and gels. Cationic cosmetic agents can include, for example, cationic conditioning agents and adhesive polymers (i.e. hair styling agents). The use of anionic surfactants can result in ionic interactions with such cationic cosmetic agents. It is therefore desirable to provide a way to emulsify the silicone gum that is essentially free of anionic surfactants.

It is the object of the present invention to formulate hair care compositions such as such as mousses, tonics, lotions, hairsprays and gels, which provide style retention and improved conditioned hair feel benefits. It is desirable to achieve improved conditioning and hair feel benefits by utilizing relatively high levels of silicone gums incorporated into hair care compositions containing a hair setting polymer. It is further desirable that these hair care compositions reduce the negative aesthetic and hair feel properties associated with hair setting polymers.

These and other benefits as may be discussed or apparent to one of ordinary skill in the art can be provided according to the invention which is described below.

All percentages herein are by weight of the composition unless otherwise indicated. All ratios are weight ratios unless otherwise indicated. Unless otherwise indicated, all percentages, ratios, and levels of ingredients referred to herein are based on the actual amount of the ingredient, and do not include solvents, fillers, or other materials with which the ingredient may be combined as commercially available products.

The invention hereof can comprise, consist of, or consist essentially of the essential elements described herein as well as any of the preferred or other optional ingredients described herein.

### SUMMARY OF THE INVENTION

The present invention comprises hair care compostions such as mousses, tonics, lotions, hairsprays and gels,

which provide style retention and improved conditioned hair feel benefits. Said compositions achieve the improved conditioning and hair feel benefits by emulsifying a silicone conditioning agent containing silicone gums. These emulsions are incorporated into hair care compositions which additionally contain a hair setting polymer or another cationic cosmetic agent; wherein the hair care compositions are essentially free of volatile solvents and contain no more than about 4.0%, by weight of the composition, of anionic surfactants, preferably no more than about 1%, more preferably no more than about 0.5%, most preferably 0%.

More specifically, the present invention provides a hair care composition that comprises:

(a) from 0.1% to 75% by weight of the composition, of an emulsified silicone conditioning agent comprising:

(i) from 20% to 100%, by weight of the silicone conditioning agent, of a nonvolatile insoluble silicone gum having a complex viscosity at 25° C of at least 1,000,000 (centistoke (CSTK)) $mm^2/S$
(ii) from 0% to 80%, by weight of the silicone conditioning agent, of a nonvolatile insoluble silicone fluid having a viscosity at 25°C of less than 1,000,000 (centistokes) $mm^2/S$

(b) from 0.1 % to 25%, by weight of the composition, of an emulsifying agent for said silicone conditioning agent;
(d) from 0% to 25%, by weight of the composition, of an additional cationic cosmetic agent.
(c) from 0.1% to 25%, by weight of the composition, of a hair setting polymer.
(e) from 24.8% to 99.7%, by weight of the composition, of an aqueous carrier;
wherein said hair care composition is essentially free of volatile solvents and said composition contains no more than about 4%, by weight of the composition, of anionic surfactants; and said composition must contain at least 0.1% of a cationic cosmetic agent selected from the group consisting of cationic surfactants or cationic polymers for providing style retention and improved conditioning.

The present invention also relates to a method for providing conditioning and style retention by applying the compositions hereof to the hair.

The compositions and methods of the present invention are advantageous for utilization in connection with a wide variety of hair care products including hair mousses, tonics, lotions, hairsprays and gels.

The present invention including various optional and preferred embodiments thereof, is described in more detail below.

## DETAILED DESCRIPTION OF THE INVENTION

The essential, as well as various optional, components of the present invention are described below.

### Non-Volatile Silicone Conditioning Agent

An essential component of the present invention is a nonvolatile silicone hair conditioning agent which is insoluble in the hair care compositions hereof. The silicone hair conditioning agent is emulsified so as to be in the form of dispersed, insoluble particles, or droplets. The silicone hair conditioning agent comprises relatively high levels of nonvolatile insoluble silicone gum and optionally a lower viscosity nonvolatile silicone fluid. The silicone gum is insoluble in the hair care composition as a whole but is soluble in the silicone fluid. The silicone hair conditioning agent can additionally comprise a silicone resin.

The silicone gum will be present in the silicone conditioning agent hereof at levels of from 20% to 100%, preferably from 35% to 80%, more preferably from 30% to 70%.

Nonvolatile insoluble silicone material that is essential in the silicone conditioning agent is silicone gum. The term "silicone gum", as used herein, means polyorganosiloxane materials having a viscosity at 25°C of at least 1,000,000 centistokes. The term "nonvolatile" as used herein shall mean that the silicone material exhibits very low or no significant vapor pressure at ambient conditions, as is understood by those in the art. Silicone gums are described in U.S. Patent 4,152,416, Spitzer et al., issued May 1, 1979 and Noll, Walter, Chemistry and Technology of Silicones, New York: Academic Press 1968. Also describing silicone gums are General Electric Silicone Rubber Product Data Sheets SE 30, SE 33, SE 54 and SE 76. The "silicone gums" will typically have a mass molecular weight in excess of about 300,000, generally between about 300,000 and about 1,000,000. Silicone gums useful herein can include polyalkyl siloxanes, polyaryl siloxanes, polyalkylaryl siloxanes, polyalkylamino siloxanes, polyether siloxanes and the like. Other insoluble, nonvolatile silicone gums having hair conditioning properties can also be used.

Silicone gums hereof include polyalkyl or polyaryl siloxanes with the following structure(I):

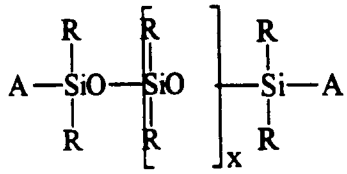

wherein R is alkyl or aryl, and x is an integer from at least 4,000, preferably from about 4,000 to about 80,000, more preferably from about 8,000 to about 40,000, most preferably from about 12,000 to about 30,000. "A" represents groups which block the ends of the silicone chains.

The alkyl or aryl groups substituted on the siloxane chain (R) or at the ends of the siloxane chains (A) may have any structure as long as the resulting silicones remain fluid at room temperature, are hydrophobic, are neither irritating, toxic nor otherwise harmful when applied to the hair, are compatible with the other components of the composition, are chemically stable under normal use and storage conditions, and are capable of being deposited on and of conditioning hair.

Suitable A groups include methyl, methoxy, ethoxy, propoxy, and aryloxy. The two R groups on the silicone atom may represent the same group or different groups. Preferably, the two R groups represent the same group. Suitable R groups include methyl, ethyl, propyl, phenyl, methylphenyl and phenylmethyl. The preferred silicones are polydimethyl siloxane, polydiethylsiloxane, and polymethylphenylsiloxane. Polydimethylsiloxane is especially preferred.

Suitable insoluble, nonvolatile silicone fluids can have the same chemical structures as the previously described silicone gums, but will be of lower viscosity, as described below, and consequently will have correspondingly lower molecular weight. The term "silicone fluid" shall mean flowable silicone materials having a viscosity of less than 1,000,000 centistokes at 25°C. Generally, the viscosity of the fluid will be between from 5 and 1,000,00 $mm^2/S$ (centistokes) at 25°C, preferably between from 10 and 600,000 $mm^2/S$ (centistokes), more preferably between from 100 and 100,000 $mm^2/S$ (centistokes). In Formula I above, the lower molecular weight silicone fluids hereof will typically have "x" values of less than 400, generally from about 8 to about 3,000.

The nonvolatile polyalkylsiloxane fluids that may be used include, for example, polydimethylsiloxanes. These siloxanes are available, for example, from the General Electric Company in their Viscasil® and SF 96 series, and from Dow Corning in their Dow Corning 200 series.

The polyalkylaryl siloxane fluids that may be used, also include, for example, polymethylphenylsiloxanes. These siloxanes are available, for example, from the General Electric Company as SF 1075 methyl phenyl fluid or from Dow Corning as 556 Cosmetic Grade Fluid.

The polyether siloxane copolymers that may be used include, for example, a polypropylene oxide modified polydimethylsiloxane (e.g., Dow Corning DC-1248) although ethylene oxide or mixtures of ethylene oxide and propylene oxide may also be used. The ethylene oxide and polypropylene oxide level must be sufficiently low to prevent solubility in water and the composition hereof.

Other references disclosing suitable silicone fluids include U.S. Patent 2,826,551, Geen; U.S. Patent 3,964,500, Drakoff, issued June 22, 1976; U.S. Patent 4,364,837, Pader; and British Patent 849,433, Woolston.

Typically, the silicone fluid will be present in the silicone conditioning agent hereof at levels of from 0% to 80%, preferably from 20% to 75%, more preferably from 30% to 70%. Specific examples of silicone fluid include polydimethylsiloxane, (polydimethylsiloxane) (methylvinylsiloxane) copolymer, poly(dimethylsiloxane) (diphenyl siloxane)(methylvinylsiloxane) copolymer and mixtures thereof.

The silicone hair conditioning agent for use herein will preferably have an average viscosity of from 1,000 to 2,000,000 $mm^2/S$ (centistokes) at 25°C, more preferably from 10,000 to 1,800,000 $mm^2/S$, even more preferably from 100,000 to 1,500,000 $mm^2/S$ . The viscosity can be measured by means of a glass capillary viscometer as set forth in Dow Corning Corporate Test Method CTM0004, July 20, 1970.

Another optional ingredient that can be included in the silicone conditioning agent is silicone resin. Silicone resins are highly crosslinked polymeric siloxane systems. The crosslinking is introduced through the incorporation of trifunctional and tetrafunctional silanes with monofunctional or difunctional, or both, silanes during manufacture of the silicone resin. As is well understood in the art, the degree of crosslinking that is required in order to result in a silicone resin will vary according to the specific silane units incorporated into the silicone resin. In general, silicone materials which have a sufficient level of trifunctional and tetrafunctional siloxane monomer units (and hence, a sufficient level of crosslinking) such that they dry down to a rigid, or hard, film are considered to be silicone resins. The ratio of oxygen atoms to silicon atoms is indicative of the level of crosslinking in a particular silicone material. Silicone materials which have at least about 1.1 oxygen atoms per silicon atom will generally be silicone resins herein. Preferably, the ratio of oxygen:silicon atoms is at least about 1.2:1.0. Silanes used in the manufacture of silicone resins include monomethyl-, dimethyl-, trimethyl-, monophenyl-, diphenyl-, methylphenyl-, monovinyl-, and methylvinyl-chlorosilanes, and tetrachlorosilane, with the methyl-substituted silanes being most commonly utilized.

Background material on scones including sections discussing silicone fluids, gums, and resins, as well as manufacture of silicones, can be found in Encyclopedia of Polymer Science and Engineering, Volume 15, Second Edition, pp 204-308, John Wiley & Sons, Inc., 1989,

Silicone materials and silicone resins in particular, can conveniently be identified according to a shorthand nomenclature system well known to those skilled in the art as "MDTQ" nomenclature. Under this system, the silicone is described according to presence of various siloxane monomer units which make up the silicone. Briefly, the symbol M denotes the monofunctional unit $(CH_3)_3SiO)$ 5; D denotes the difunctional unit $(CH_3)_2SiO$; T denotes the trifunctional unit $(CH_3)SiO_{1.5}$ ; and Q denotes the quadri- or tetra-functional unit $SiO_2$. Primes of the unit symbols, e.g., M', D', T', and Q' denote substituents other than methyl, and must be specifically defined for each occurrence. Typical alternate substituents include groups such as vinyl, phenyls, amines, hydroxyls, etc. The molar ratios of the various units, either in terms of subscripts to the symbols indicating the total number of each type of unit in the silicone (or an average thereof) or as specifically indicated ratios in combination with molecular weight complete the description of the silicone material under the MDTQ system. Higher relative molar amounts of T, Q, T' and/or Q' to D, D', M and/or or M' in a silicone resin is indicative of higher levels of crosslinking. As discussed before, however, the overall level of crosslinking can also be indicated by the oxygen to silicon ratio.

The silicone resins for use herein which are preferred are MQ, MT, MTQ, MQ and MDTQ resins. Thus, the preferred silicone substituent is methyl. Especially preferred are MQ resins wherein the M:Q ratio is from about 0.5:1.0 to about 1.5:1.0 and the average molecular weight of the resin is from about 1000 to about 10,000.

If the silicone resin is present in said compositions, it will typically be present at a level of from about 0.5% to about 35%, preferably from about 3% to about 30%, more preferably from about 5% to about 20% of the silicone conditioning agent.

Emulsifying Agent

The hair care composition according to the invention comprise as an essential component an emulsifying agent. The emulsifying agent hereof is selected from nonionic surfactants, cationic surfactants, amphoteric surfactants, zwitterionic surfactants and mixtures thereof. Preferably, the emulsifying agent is a nonionic or cationic surfactant, more preferably, a nonionic surfactant.

Nonionic surfactants which are useful in the present invention can be broadly defined as including compounds containing an alkylene oxide groups (hydrophilic in nature) with a hydrophobic compound, which may be aliphatic or alkyl aromatic in nature. Examples of preferred classes of nonionic surfactants are:

1. The polyethylene oxide condensates of alkyl phenols, e.g., the condensation products of the alkyl phenols having an alkyl group containing from about 6 to about 20 carbon atoms in either a straight chain or branched chain configuration, with ethylene oxide, the said ethylene oxide being present in amounts equal to from about 3 to about 60 moles of ethylene oxide per mole of alkyl phenol.
2. Those derived from the condensation of ethylene oxide with the product resulting from the reaction of propylene oxide and ethylene diamine products.
3. The condensation product of aliphatic alcohols having from about 8 to about 18 carbon atoms, in either straight chain or branched chain configuration, with ethylene oxide, e.g., a coconut alcohol ethylene oxide condensate having from about 2 to about 30 moles of ethylene oxide per mole of coconut alcohol, the coconut alcohol fraction having from about 10 to about 14 carbon atom.
4. Long chain tertiary amine oxides such as those corresponding to the following general formula:

$$R_1R_2R_3N \rightarrow O$$

wherein $R_1$ contains an alkyl, alkenyl or monohydroxy alkyl redical of from about 8 to about 18 carbon atoms, from 0 to about 10 ethylene oxide moieties, and from 0 to about 1 glyceryl moiety, and $R_2$ and $R_3$ contain from about 1 to about 3 carbon atoms and from 0 to about 1 hydroxy group, e.g., methyl, ethyl, propyl, hydroxyethyl, or hydroxypropyl radicals (the arrow in the formula represents a semipolar bond).

5. Long chain tertiary phosphine oxides corresponding to the following general formula:

$$RR'R''P \rightarrow O$$

wherein R contains an alkyl, alkenyl or monohydroxyalkyl radical ranging from about 8 to about 18 carbon atoms in chain length, from 0 to about 10 ethylene oxide moieties and from 0 to about 1 glyceryl moiety and R' and R'' are

each alkyl or monohydroxyalkyl groups containing from about 1 to about 3 carbon atoms. The arrow in the formula represents a semipolar bond.

6. Long chain dialkyl sulfoxides containing one short chain alkyl or hydroxy alkyl radical of from about 1 to about 3 carbon atoms (usually methyl) and one long hydrophobic chain which include alkyl, alkenyl, hydroxy alkyl, or keto alkyl radicals containing from about 8 to about 20 carbon atoms, from 0 to about 10 ethylene oxide moieties and from 0 to about 1 glyceryl moiety.

7. Polysorbates, e.g., sucrose esters of fatty acids. Such materials are described in U.S. Patent 3,480,616, e.g., sucrose cocoate (a mixture of sucrose esters of a coconut acid, consisting primarily of monoesters, and sold under the tradenames GRILLOTEN LSE 87K from RITA, and CRODESTA SL-40 from Croda).

8. Alkyl polysaccharide nonionic surfactants are disclosed in U.S. Patent 4,565,647, Llenado, issued January 21, 1986, having a hydrophobic group containing from about 6 to about 30 carbon atoms, preferably from about 10 to about 16 carbon atoms and a polysaccharide, e.g., a polyglycoside, hydrophilic group. The polysaccharide can contain from about 1.0 to about 10, preferably from about 1.3 to about 3, most preferably from about 1.3 to about 2.7 saccharide units. Any reducing saccharide containing 5 or 6 carbon atoms can be used, e.g., glucose, galactose and galactosyl moieties can be substituted for the glucosyl moieties. (Optionally the hydrophobic group is attached at the 2-,3-, 4-, etc. positions thus giving a glucose or galactose as opposed to a glucoside or galactoside.) The intersaccharide bonds can be, e.g., between the one position of the additional saccharide units and the 2-, 3-, 4-, and/or 6-positions on the preceding saccharide units. Optionally there can be a polyalkyleneoxide chain joining the hydrophobic moiety and the polysaccharide moiety. The alkyl group preferably contains up to about 3 hydroxy groups and/or the polyalkyleneoxide chain can contain up to about 10, preferably less than 5, alkylene moieties. Suitable alkyl polysaccharides are octyl, nonyldecyl, undecyldodecyl, tridecyl, tetradecyl, pentadecyl, hexadecyl, heptadecyl, and octadecyl, di-, tri-, tetra-, penta-, and hexaglucosides, galactosides, lactosides, glucoses, fructosides, fructoses and/or galactoses.

9. Polyethylene glycol (PEG) glyceryl fatty esters, as depicted by the formula $RC(O)OCH_2CH(OH)CH_2(OCH_2CH_2)_nOH$ wherein n is from about 5 to about 200, preferably from about 20 to about 100, more preferably from about 30 to about 85, and RC(O)- is an ester wherein R comprises an aliphatic radical having from about 7 to 19 carbon atoms, preferably from about 9 to 17 carbon atoms, more preferably from about 11 to 17 carbon atoms, most preferably from about 11 to 14 carbon atoms. The combinations of n from about 20 to about 100, with $C_{12}$-$C_{18}$, preferably $C_{12}$-$C_{15}$ fatty esters, for minimized adverse effect on foaming, is preferred.

Cationic surfactants useful in hair care compositions of the present invention, contain amino or quaternary ammonium hydrophilic moieties which are positively charged when dissolved in the aqueous composition of the present invention. Cationic surfactants among those useful herein are disclosed in the following documents: M.C. Publishing Co., McCutcheon's Detergents & Emulsifiers, (North American edition 1979); Schwartz, et al., Surface Active Agents, Their Chemistry and Technology, New York: Interscience Publishers, 1949; U.S. Patent 3,155,591, Hilfer, issued November 3, 1964; U.S. Patent 3,929,678, Laughlin, et a., issued December 30, 1975; U.S. Patent 3,959,461, Bailey, et al., issued May 25, 1976; and U.S. Patent 4,387,090, Bolich, Jr., issued June 7, 1983.

Among the quaternary ammonium-containing cationic surfactant materials useful herein are those of the general formula:

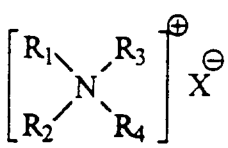

wherein $R_1$-$R_4$ are independently an aliphatic group of from about 1 to about 22 carbon atoms, or an aromatic, alkoxy, polyoxyalkylene, alkylamido, hydroxyalkyl, aryl or alkylaryl group having from about 12 to about 22 carbon atoms; and X is an anion selected from halogen, acetate, phosphate, nitrate and alkylsulfate radicals. The aliphatic groups may contain, in addition to carbon and hydrogen atoms, ether linkages, and other groups such as amino groups. The longer chain aliphatic groups, eg., those of about 12 carbons, or higher, can be saturated or unsaturated.

Salts of primary, secondary and tertiary fatty amines are also suitable cationic surfactant materials. The alkyl groups of such amines preferably have from about 12 to about 22 carbon atoms, and may be substituted or unsubstituted. Such amines, useful herein, include stearamido propyl dimethyl amine, diethyl amino ethyl stearamide, dimethyl stearamine, dimethyl soyamine, soyamine, myristyl amine, tridecyl amine, ethyl stearylamine, N-tallowpropane diamine, ethoxylated (5 moles E.O.) stearylamine, dihydroxy ethyl stearylamine, and arachidylbehenylamine. Suitable amine salts include the halogen, acetate, phosphate, nitrate, citrate, lactate and alkyl sulfate salts. Such salts include stearylamine hydrochloride, soyamine chloride, stearylamine formate, N-tallowpropane diamine dichloride and stearamidopropyl dimethylamine citrate. Cationic amine surfactants included among those useful in the present invention are disclosed in U.S. Patent 4,275,055, Nachtigal, et al., issued June 23, 1981,

Examples of amphoteric surfactants which can be used in the compositions of the present invention are those which are broadly described as derivatives of aliphatic secondary and tertiary amines in which the aliphatic radical can be straight or branched chain and wherein one of the aliphatic substituents contains from about 8 to about 18 carbon atoms and one contains an anionic water solubilizing group, e.g., carboxy, sulfonate, sulfate, phosphate, or phosphonate. Examples of compounds falling within this definition are sodium 3-dodecyl-aminopropionate, sodium 3-dodecylaminopropane sulfonate, N-alkyltaurines such as the one prepared by reacting dodecylamine with sodium isethionate according to the teaching of U.S. Patent 2,658,072, N-higher alkyl aspartic acids such as those produced according to the teaching of U.S. Patent 2,438,091, and the products sold under the trade name "Miranol" and described in U.S. Patent 2,528,378.

Zwitterionic surfactants, useful in the present invention, are exemplified by those which can be broadly described as derivatives of aliphatic quaternary ammonium, phosphonium and sulfonium compounds, in which the aliphatic radicals can be straight or branched chain, and wherein one of the aliphatic substituents contains from about 8 to about 18 carbon atoms and one contains an anionic water-solubilizing group, e.g., carboxy, sulfonate, sulfate, phosphate, or phosphonate. A general formula for these compounds is:

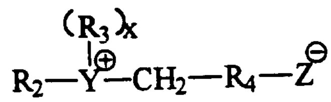

wherein $R_2$ contains an alkyl, alkenyl, or hydroxy alkyl radical of from about 8 to about 18 carbon atoms, from 0 to about 10 ethylene oxide moieties and from 0 to about 1 glyceryl moiety; Y is selected from the group consisting of nitrogen, phosphorus, and sulfur atoms; $R_3$ is an alkyl or monohydroxyalkyl group containing about 1 to about 3 carbon atoms; X is 1 when Y is a sulfur atom, and 2 when Y is a nitrogen or phosphorus atom; $R_4$ is an alkylene or hydroxyalkylene of from about 1 to about 4 carbon atoms and Z is a radical selected from the group consisting of carboxylate, sulfonate, sulfate, phosphonate, and phosphate groups.

Other zwitterionics such as betaines are also useful in the present invention. Examples of betaines useful herein include the high alkyl betaines, such as coco dimethyl carboxymethyl betaine, lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, and lauryl bis-(2-hydroxypropyl)alpha-carboxyethyl betaine. The sulfobetaines may be represented by coco dimethyl sulfopropyl betaine, stearyl dimethyl sulfopropyl betaine, lauryl dimethyl sulfoethyl betaine, lauryl bis-(2-hydroxyethyl) sulfopropyl betaine and the like; amidobetaines and amidosulfobetaines, wherein the $RCONH(CH_2)_3$ radical is attached to the nitrogen atom of the betaine are also useful in this invention.

Other surfactants that can be used include fluorosurfactants, or other halogenated surfactants, which can be nonionic, cationic, amphoteric, or zwitterionic.

Fluorosurfactants include perfluorinated compounds such as those represented by the formula

$$CF_3-(CF_2)_x-(CH_2)_y-Z$$

where Z is a water solubilizing group of either organic or inorganic character, x is an integer which is generally from 2 to 17, particularly from 7 to 11, and y is an integer from 0 to 4, and said compounds may be nonionic, cationic, amphoteric or zwitterionic, depending upon the nature of the grouping or groupings encompassed by Z. The Z groups may be or may comprise sulfate, sulfonate, carboxylate, amine salt, quaternary ammonium, phosphate, phosphonate, and combinations thereof. The perfluorinated compounds are known in the art. These compounds are described in U.S. Patent 4,176,176, Cella et al., issued November 27, 1979; U.S. Patent 3,993,745, Cella et al., issued November 23, 1976, and U.S. Patent 3,993,744, Cella et al., issued November 23, 1976. Fluorosurfactants, when used, will typically be used at lower levels than most other emulsifying agents.

Another surfactant which can be present in the invention are polyether siloxane copolymers, or silicone "copolyols" as they are sometimes referred to. Silicone copolyols are surfactants charecterized by a hydrophobic polysiloxane chain and a hydrophilic alkoxy portion.

Silicone copolyols which may be used include polyalkylene oxide modified polydimethylsiloxanes of the following formulae:

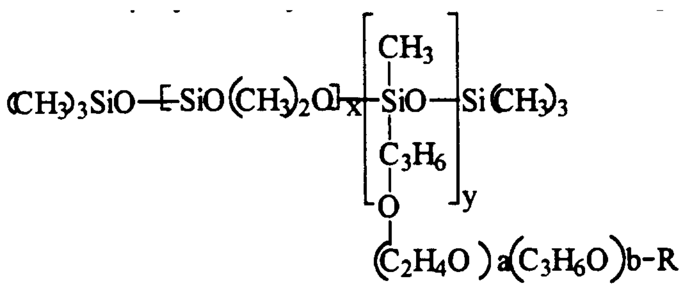

and

$$R'-Si\left[[OSi(CH_3)_2]x-(OC_2H_4)a-(OC_3H_6)b-OR''\right]_3$$

wherein R is hydrogen, an alkyl group having from 1 to about 12 carbon atoms, an alkoxy group having from 1 to about 6 carbon atoms or a hydroxyl group; R' and R" are alkyl groups having from 1 to about 12 carbon atoms; x is an integer of from 1 to 100, preferably from 20 to 30; y is an integer of 1 to 20, preferably from 2 to 10; and a and b are integers of from 0 to 50, preferably from 20 to 30.

Silicone copolyols among those useful herein are also disclosed in the following patent documents, U.S. Patent 4,122,029, Geen, et al., issued October 24, 1978; U.S. Patent 4,265,878, Keil, issued May 5, 1981; and U.S. Patent 4,421,769, Dixon, et al., issued December 20, 1983. Such silicone copolyol materials are also disclosed, in hair compositions, in British Patent Application 2,066,659, Abe, published July 15, 1981 and Canadian Patent 727,588, Kuehns, issued February 8, 1966. Commercially available silicone copolyols which can be used herein, include Silwet Surface Active Copolymers (manufactured by the Union Carbide Corporation); and Dow Corning Silicone Surfactants (manufactured by the Dow Corning Corporation).

Emulsion

The hair care composition according to the invention comprises an aqueous emulsion of the silicone conditioning agent. The emulsion of the present invention is prepared by combining the silicone gum in the optional silicone fluid, adding the emulsifying agent and forming the emulsion with the addition of the aqueous carrier (which is described in further detail below) and mixing to form an emulsion..

The silicone conditioning agent is present in the hair care compositions in an amount of from 0.1% to 75%, preferably from 0.5% to 50%, more preferably from 1% to about 15%, most preferably from 2% to 10%. The weight ratio of the emulsifying agent to emulisfy the silicone conditioning agent, as described above, to silicone conditioning agent is preferably from about 1:25, preferably from about 2:1, more preferably from about 1:10, most preferably from about 1:1.

The emulsified particles of the silicone conditioning agent, will preferably have an average diameter of less than 25μm, more preferably from about 0.1 to about 10 μm. The average particle size may be measured for example by laser light scattering technique such as a Particle Size Analyzer from Malvern Instruments.

Hair Setting Polymers

The hair care compositions of the present invention contain, as an essential component a hair setting polymer. Any hair setting polymer soluble or dispersible in the aqueous carrier may be used. Solubility is determined at ambient conditions (e.g. temperature about 25°C and atmospheric pressure). Hair setting polymers provide style retention benefits upon application to the hair and drying to form an adhesive film. A wide variety of hair setting polymers are known in the art. Suitable types of hair setting polymers include anionic, nonionic, amphoteric, and cationic. Prefered hair setting polymers are cationic or amphoteric, especially preferred are cationic hair setting polymer. Specific hair setting polymers include: vinyl pyrrolidone/vinyl acetate copolymers; vinylacetate homopolymer; t-butyl acrylate homopolymer; t-butyl styrene/ethyl hexyl methacrylate copolymer; dimethyl acrylamide/ t-butyl acrylate/ethyl hexyl methacrylate copolymer; ethylene/vinyl acetate copolymer; allyl alcohol/styrene copolymer; vinyl chloride/vinyl acetate copolymer; vinyl pyrrolidone/vinyl acetate/butyl acrylate copolymer; vinyl pyrrolidone/vinyl acetate/butyl acrylate/stryrene sulfonate copolymer; vinyl pyrrolidone/vinyl propionate copolymer; vinyl caprolactam/vinyl acetate copoylmer; and styling resins sold under the trade names Ultrahold CA 8® by Ciba Geigy (ethyl acrylate/ acrylic acid/N-t-butyl acrylamide copolymer); Resyn 28-1310® by National Starch and Luviset CA 66® by BASF (vinyl acetate/reotonic acid copolymer 90/10); Luviset CAP® by BASF (vinyl acetate/vinyl propionate/crotonic acid); Resyn 28-2930® by National Starch (vinyl acetate/vinyl neodecanoate/crotonic acid copolymer); Gantrez AN® by GAF (polyvinyl methyl ether/maleic anhydride copolymer); AMPHOMER® by National Starch (octylacrylamide/acrylate/butylaminoethyl methacrylate copolymer); Celquat H-100 and Celquat L200 by National Starch (diallyldimonium/hydroxyethycellulose copolymer), and mixtures thereof.

With certain of the polymers it may be desirable to neutralize some acidic groups to promote solubility (e.g. PVA/crotonic acid). Examples of suitable neutralizing agents include 2-amino-2-methyl-1,3 propanediol (AMPD); 2-amino-2-ethyl-1,3-propanediol (AEPD); 2-amino-2-methyl-1-propanol (AMP); 2-amino-1-butanol (AB); monoethanolamine (MEA); diethanolamine (DEA); triethanolamine (TEA); monoisopropanolamine (MIPA); diisopropanol amine (DIPA); triisopropanolamine (TIPA); and dimethyl stearamine (DMS).

The hair setting polymer is present in the hair care compositions of the present invention at a level of from 0.1% to 25%, preferably from 0.5% to 15%, more preferably from 1% to 10% by weight of the composition.

Cationic Cosmetic Agent

The compositions of the present invention must contain a cationic cosmetic agent or cationic emulsifier. The cationic cosmetic agent may be a cationic hair setting polymer or a cationic emulsifier if present at a sufficient level to provide a conditioning benefit, as described above. However, if neither the hair setting agent nor the emulsifer is cationic, the compositons must contain one or more other cationic cosmetic agents. Such additional cationic cosmetic agents useful in the present invention can be any cationic material which provides a cosmetic benefit to the hair ( e.g., conditioning, shine, hair coloring, and the like). Preferably such cationic materials are selected from the group consisting of cationic surfactants, cationic polymers, and mixtures thereof. These cationic materials can also be persent in combination with cationic hair setting polymers and/or cationic emulsifiers.

Thus, the compositions hereof will contain an additional cationic cosmetic agent at a level of from 0% to 25%, by weight of the composition. If the composition does not contain a cationic hair setting polymer or a cationic emulsifier, as described above, such composition will comprise from 0.1% to 25% of one or more other types of cationic cosmetic agents, preferably from 0.1% to 15%, more preferably from 0.5% to 10%. In the event that the composition does contain cationic hair setting polymer or emulsifer, such components are only to be included in the calculation of hair setting polymer or emulsifier, respectively, and are not to be included as part of the additional cationic cosmetic agent. Additionally, the emulsifier component as described above includes only that type and amount of surfactant that is used to emulsify the silicone conditioning agent. Cationic surfactant that is added or used otherwise (such as after the emulsion is formed) would constitute part of the additional cationic cosmetic agent.

Cationic surfactants, as described previously, are used to provide additional conditioning benefits in the present compositions. The cationic surfactants used herein can be used at higher level than the cationic surfactants used as an emulsifying agent. Preferably, the cationic surfactants of the present invention contain amino or quaternary ammonium moieties. Cationic surfactants among those useful herein are disclosed in the following documents, M.C. Publishing Co., McCutcheon's, Detergents & Emulsifiers, (North American edition 1979); Schwartz, et al., Surface Active Agents, Their Chemistry and Technology, New York: Interscience Publishers, 1949; U.S. Patent 3,155,591 , Hilfer issued November 3, 1964; U. S. Patent 3,929,678, Laughlin et al., issued December 30, 1975; U. S. Patent 3,959,461, Bailey et al., issued May 25, 1976; and U. S. Patent 4,387,090, Bolich, Jr., issued June 7, 1983.

The compositions of the present invention can also comprise one or more cationic polymers. The cationic polymers used in the present invention can provide both conditioning and styling benefits. The cationic polymer will preferably be water soluble. Cationic polymers are typically used in the same ranges as disclosed above for cationic surfactants.

By "water soluble" cationic polymer, what is meant is a polymer which is sufficiently soluble in water to form a substantially clear solution to the naked eye at a concentration of 0.1% in water (distilled or equivalent) at 25° C. Preferably, the polymer will be sufficiently soluble to form a substantially clear solution at 0.5% concentration, more preferably at 1.0% concentration.

As used herein, the term "polymer" shall include materials whether made by polymerization of one type of monomer or made by two (i.e., copolymers) or more types of monomers.

The cationic polymers hereof will generally have a weight average molecular weight which is at least about 5,000, typically at least about 10,000, and is less than about 10 million. Preferably, the molecular weight is from about 100,000 to about 2 million. The cationic polymers will generally have cationic nitrogen-containing moieties such as quaternary ammonium or cationic amino moieties, or a mixture thereof.

Any anionic counterions can be utilized for the cationic polymers so long as the water solubility criteria is met. Suitable counterions include halides (e.g., Cl, Br, I, or F, preferably Cl, Br, or I), sulfate, and methylsulfate. Others can also be used, as this list is not exclusive.

The cationic nitrogen-containing moiety will be present generally as a substituent, on a fraction of the total monomer units of the cationic hair conditioning polymers. Thus, the cationic polymer can comprise copolymers, terpolymers, etc. of quaternary ammonium or cationic amine-substituted monomer units and other non-cationic units referred to

herein as spacer monomer units. Such polymers are known in the art, and a variety can be found in the CTFA Cosmetic Ingredient Dictionary, 3rd edition, edited by Estrin, Crosley, and Haynes, (The Cosmetic, Toiletry, and Fragrance Association, Inc., Washington, D.C., 1982).

Suitable cationic polymers include, for example, copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. The alkyl and dialkyl substituted monomers preferably have $C_1$ -$C_7$ alkyl groups, more preferably $C_1$-$C_3$ alkyl groups. Other suitable spacer monomers include vinyl esters, vinyl alcohol (made by hydrolysis of polyvinyl acetate), maleic anhydride, propylene glycol, and ethylene glycol.

The cationic amines can be primary, secondary, or tertiary amines, depending upon the particular species and the pH of the shampoo. In general, secondary and tertiary amines, especially tertiary amines, are preferred.

Amine-substituted vinyl monomers can be polymerized in the amine form, and then optionally can be converted to ammonium by a quaternization reaction. Amines can also be similarly quaternized subsequent to formation of the polymer. For example, tertiary amine functionalities can be quaternized by reaction with a salt of the formula R'X wherein R' is a short chain alkyl, preferably a $C_1$-$C_7$ alkyl, more preferably a $C_1$-$C_3$ alkyl, and X is an anion which forms a water soluble salt with the quaternized ammonium.

Suitable cationic amino and quaternary ammonium monomers include, for example, vinyl compounds substituted with dialkylaminoalkyl acrylate, dialkylaminoalkyl methacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidone, e.g., alkyl vinyl imidazolium, alkyl vinyl pyridinium, alkyl vinyl pyrrolidone salts. The alkyl portions of these monomers are preferably lower alkyls such as the $C_1$-$C_3$ alkyls, more preferably $C_1$ and $C_2$ alkyls. Suitable amine-substituted vinyl monomers for use herein include dialkylaminoalkyl acrylate, dialkylaminoalkyl methacrylate, dialkylaminoalkyl acrylamide, and dialkylaminoalkyl methacrylamide, wherein the alkyl groups are preferably $C_1$ -$C_7$ hydrocarbyls, more preferably $C_1$-$C_3$, alkyls.

The cationic polymers hereof can comprise mixtures of monomer units derived from amine- and/or quaternary ammonium-substituted monomer and/or compatible spacer monomers.

Suitable cationic hair conditioning polymers include, for example: copolymers of 1-vinyl-2-pyrrolidone and 1-vinyl-3-methylimidazolium salt (e.g., chloride salt) (referred to in the industry by the Cosmetic, Toiletry, and Fragrance Association, "CTFA", as Polyquaternium-16), such as those commercially available from BASF Wyandotte Corp. (Parsippany, NJ, USA) under the LUVIQUAT tradename (e.g., LUVIQUAT FC 370); copolymers of 1-vinyl-2-pyrrolidone and dimethylaminoethyl methacrylate (referred to in the industry by CTFA as Polyquaternium-11) such as those commercially available from Gaf Corporation (Wayne, NJ, USA) under the GAFQUAT tradename (e.g., GAFQUAT 755N); cationic diallyl quaternary ammonium-containing polymers, including, for example, dimethyldiallylammonium chloride homopolymer and copolymers of acrylamide and dimethyldiallylammonium chloride, referred to in the industry (CTFA) as Polyquaternium 6 and Polyquaternium 7, respectively; and mineral acid salts of amino-alkyl esters of homo- and copolymers of unsaturated carboxylic acids having from 3 to 5 carbon atoms, as described in U.S. Patent 4,009,256.

Other cationic polymers that can be used include polysaccharide polymers, such as cationic cellulose derivatives and cationic starch derivatives.

Cationic polysaccharide polymer materials suitable for use herein include those of the formula:

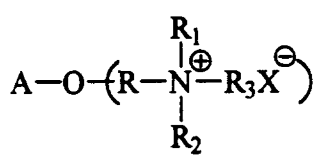

wherein: A is an anhydroglucose residual group, such as a starch or cellulose anhydroglucose residual, R is an alkylene oxyalkylene, polyoxyalkylene, or hydroxyalkylene group, or combination thereof, $R_1$, $R_2$, and $R_3$ independently are alkyl, aryl, alkylaryl, arylalkyl, alkoxyalkyl, or alkoxyaryl groups, each group containing up to about 18 carbon atoms, and the total number of carbon atoms for each cationic moiety (i.e., the sum of carbon atoms in $R_1$, $R_2$ and $R_3$) preferably being about 20 or less, and X is an anionic counterion, as previously described.

Cationic cellulose is available from Amerchol Corp. (Edison, NJ, USA) in their Polymer JR® and LR® series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted opoxide, referred to in the industry (CTFA) as Polyquaternium 24. These materials are available from Amerchol Corp. (Edison, NJ, USA) under the tradename Polymer LM-200.

Other cationic polymers that can be used include cationic guar gum derivatives, such as guar hydroxypropyltrimonium chloride (commercially available from Celanese Corp. in their JaguarR series). Other materials include quaternary nitrogen-containing cellulose ethers (e.g., as described in U.S. Patent 3,962,418, and copolymers of etherified cellulose and starch (e.g., as described in U.S. Patent 3,958,581.

Aqueous Carrier

The present hair care compositions also comprise an aqueous carrier. This can comprise any of those aqueous carriers conventionally used in hair care compositions. Generally, water is present in the aqueous carrier of the present hair care compositions of from about 50% to about 99.7%, preferably of from about 65% to about 95%, more preferably of from about 80% to about 95%, by weight of the composition. Optionally, other water miscible fluids can also be used in the aqueous carrier such as, (but not limited to) ethanol, propanol, and the like.

Compositions of the present invention contain no more than about 4%, by weight of the composition, of an anionic surfactant, preferably no more than about 1%, more preferably no more than 0.5%, most preferably no more than 0.1%. Additionally, said compositions will contain no more than about 2%, by weight of the composition, of volatile solvents (e.g. cyclomethicone), perferably no more than about 0.5%, more preferably no more than about 0.2%, most preferably no more than about 0.1%.

Thickening Agents

Hair care compositions can additionally include thickening agents or viscocity modifiers. Such thickening agents and viscosity modifiers are generally described in the following documents : Barry, "The Self Bodying Action of the Mixed Emulsifier Sodium Dodecyl Sulfate/Cetyl Alcohol", 28 *J. of Colloid and Interface Science* 82-91 (1968); Barrey, et al., "The Self-Bodying Action of Alkyltrimethylammonium Bromides/-Cetostearyl Alcohol Mixed Emulsifiers; Influence of Quaternary Chain Length", 35 *J. of Calloid and Interface Science* 689-708 (1971); and Barry, et al., "Rheology of Systems Containing Cetomacrogol 1000 Cetostearyl Alcohol, I. Self Bodying Action", 38 *J. of Colloid and Interface Science* 616-635 (1972).

The hair care compositions of the present invention may incorporate one or more lipid materials (herein referred to as comprising a "lipid vehicle material", singly or in combination), which are essentially water-insoluble. Lipid vehicle materials include naturally or synthetically-derived acids, acid derivatives, alcohols, esters, ethers, ketones, and amides with carbon chains from 12 to 22, preferably from 16 to 18, carbon atoms in length. Fatty esters are preferred; fatty alcohols are particularly preferred.

Lipid vehicle materials among those useful herein are diclosed in *Bailey's Industrial Oil and Fat Products,* (3rd edition, D. Swern, ed. 1979). Fatty alcohols included among those useful U.S. Pat. No. 3,155,591, Hilfer, issued Nov. 3, 1964; U.S. Pat. No. 4,165,369, Watanabe, et al., issued Aug. 21, 1979; U.S. Pat. No. 4,269,824, Villamarin, et al., issued May 26, 1981; *British Specification* No. 1,532,585, published Nov. 15, 1978; and Fukushima, et al., " The Effect of Cetostearyl Alcohol in Cosmetic Emulsions", 98 *Cosmetics & Toiletries* 89-112 (1983). Fatty eaters included among those useful herein are disclosed in U.S. Pat. No. 3,341,465 Kaufman, et al., issued Sept. 12, 1976. If included in the hair care compositions of the present invention, the lipid vehicle material is present at a level of from about 0.1% to about 10.0%.

Another type of thickening agent that can be used are cationic crosslinked polymers. Suitable crosslinked polymers for use herein are generally described in U.S. Patent 5,100,660, to Hawe et al., issued March 31, 1992; U.S. Patent 4,849,484, to Heard, issued July 18, 1989; U.S. Patent 4,835,206, to Farrar et al., issued May 30, 1989; U.S. Patent 4,628,078 to Glover et al. issued December 9, 1986; U.S. Patent 4,599,379 to Flesher et al. issued July 8, 1986; and EP 228,868, to Farrar et al., published July 15, 1987; The polymers hereof can be characterized by the general formula: $(A)_m(B)_n(C)_p$ wherein (A) is a dialkylaminoalkyl acrylate monomer or its quaternary ammonium or acid addition salt, (B) is a dialkylaminoalkyl methacrylate monomer or its quaternary ammonium or acid addition salt, (C) is a nonionic monomer that is polymerizable with (A) or (B) having a carbon-carbon double bond, that is polymerizable with (A) or (B), m is an integer of 0 or greater, n is an integer of 0 or greater, either m or n, or both, must be 1 or greater, and p is an integer of 0 or greater.

The (C) monomer can be selected from any of the commonly used monomers. Nonlimiting examples of these monomers include ethylene, propylene, butylene, isobutylene, eicosene, maleic anhydride, acrylamide, methacrylamide, maleic acid, acrolein, cyclohexene, ethyl vinyl ether, and methyl vinyl ether. In the cationic polymers of the present invention, (C) is preferably acrylamide. The alkyl portions of the (A) and (B) monomers are short chain length alkyls such as $C_1$-$C_8$, preferably $C_1$-$C_5$, more preferably $C_1$-$C_3$, most preferably $C_1$-$C_2$. When quaternized, the polymers are preferably quarternized with short chain alkyls, i.e., $C_1$-$C_8$, preferably $C_1$-$C_5$, more preferably $C_1$-$C_3$, most preferably $C_1$-$C_2$. The acid addition salts refer to polymers having protonated amino groups. Acid addition salts can be performed through the use of halogen (e.g. chloride), acetic, phosphoric, nitric, citric, or other acids.

These $(A)_m(B)_n(C)_p$ polymers also contain a crosslinking agent, which is typically a material containing two or more unsaturated functional groups. The crosslinking agent is reacted with the monomer units of the polymer and is incorporated into the polymer, forming either links or covalent bonds between two or more individual polymer chains or between two or more sections of the same polymer chain. Nonlimiting examples of suitable crosslinking agents include those selected from methylenebisacrylamides, diacrylates, dimethacrylates, di-vinyl aryl (e.g. di-vinyl phenyl ring) compounds, polyalkenyl polyethers of polyhydric alcohols, allyl acrylates, vinyloxyalkylacrylates, and polyfunctional vinylidenes. Specific examples of crosslinking agents useful herein include those selected from the group consisting of methylenebisacrylamide, ethylene glycol, propylene glycol, butylene glycol, di-(meth)acrylate, di-(meth)acrylamide, cyanomethylacrylate, vinyloxyethylacrylate, vinyloxyethylmethacrylate, allyl pentaerythritol, trimethylolpropane diallylether, ally sucrose, butadiene, isoprene, 1,4 di-ethylene benzene, divinyl naphthalene, ethyl vinyl ether, methyl vinyl ether, and allyl acrylate. Other crosslinkers include formaldehyde and glyoxal. Preferred for use herein as a crosslinking agent is methylenebisacrylamide.

Widely varying amounts of the crosslinking agent can be employed depending upon the properties desired in the final polymer, e.g. viscosifying effect. The crosslinking agent will typically comprise from about 1 ppm to about 10,000 ppm, preferably from about 5 ppm to about 750 ppm, more preferably from about 25 ppm to about 500 ppm, even more preferably from about 100 ppm to about 500 ppm, and most preferably from about 250 ppm to about about 500 ppm of the total weight of the polymer on a weight/weight basis.

An example of such a cationic polymer is one that is commercially available as a mineral oil dispersion (which can also include various dispersing aids such as PPG-1 trideceth-6) under the trademark Salcare$^R$ SC92 from Allied Colloids Ltd. (Norfolk, Virginia). This polymer has the proposed CTFA designation, "Polyquaternium 32 (and) Mineral Oil". Another example of such a polymer is commercially available as a mineral oil dispersion also containing PPG-1 trideceth-6 as a dispersing aid, from Allied Colloids Ltd, (Norfolk, VA) under the trademark Salcare$^R$ SC95. This product has been designated by the Cosmetics, Toiletries, and Fragrance Association (CTFA) as "Polyquatemium 37 (and) Mineral Oil (and) PPG-1 Trideceth-6".

Other thickening agents useful herein are characterized by the general formula:

$$H(OCH_2CH_2)_nOH \text{ or } H(OCH_2(CH_3)CH)_nOH$$

wherein n has an average value of from about 2,000 to about 14,000, preferably from about 5,000 to about 9,000, more preferably from about 6,000 to about 8,000.

Other polymers useful herein are PEG-2M wherein x equals 2 and n has an average value of about 2,000 (PEG 2-M is also known as Polyox WSR® N-10 from Union Carbide and as PEG-2,000); PEG-5M wherein x equals 2 and n has an average value of about 5,000 (PEG 5-M is also known as Polyox WSR® N-35 and Polyox WSR® N-80, both from Union Carbide and as PEG-5,000 and Polyethylene Glycol 300,000); PEG-7M wherein x equals 2 and n has an average value of about 7,000 (PEG 7-M is also known as Polyox WSR® N-750 from Union Carbide); PEG-9M wherein x equals 2 and n has an average value of about 9,000 (PEG 9-M is also known as Polyox WSR® N-3333 from Union Carbide); and PEG-14M wherein x equals 2 and n has an average value of about 14,000 (PEG 14-M is also known as Polyox WSR® N-3000 from Union Carbide.)

Preferred esters for use herein include cetyl palmitate and glycerylmonostearate. Cetyl alcohol an stearyl alcohol are preferred alcohols. A particularly preferred lipid vehicle material is comprised of a mixture of cetyl alcohol and stearyl alcohol containing from about 55% to about 65% (by weight of mixture) of cetyl alcohol.

Hair care compositions for which the present invention can be useful include, for example, mousses, tonics, lotions, hairsprays, and gels. Tonics, gels and non-aerosol hair sprays utilize a solvent such as water or alcohol while mousses and aerosol hair sprays additionally utilize a propellant such as trichlorofluoromethane, dichlorodifluoromethane, difluoroethane, dimethylether, propane, n-butane or isobutane. The level of propellant can be adjusted as desired but is generally from about 3% to about 30% of mousse compositions and from about 15% to about 50% of the aerosol hair spray compositions.

Suitable spray containers are well known in the art and include conventional, non-aerosol pump sprays i.e., "atomizers," aerosol containers or can having propellant, as described above, and also pump aerosol containers utilizing compressed air as the propellent. Pump aerosol containers are disclosed, for example, in U.S. Patents 4,077,441, March 7, 1978, Olofsson and 4,850,577, July 25, 1989, TerStege and also in U.S. Serial No. 07/839,648, Gosselin, Lund, Sojka, and Lefebvre, filed February 21, 1992, "Consumer Product Package Incorporating A Spray Device Utilizing Large Diameter Bubbles. Pump aerosols hair sprays using compressed air are also currently marketed by The Procter & Gamble Company under their tradename VIDAL SASSOON AIRSPRAY® hair sprays.

Optional Ingredients

The compositions herein can contain a variety of other optional components suitable for rendering such compositions more cosmetically or aesthetically acceptable or to provide them with additional usage benefits. Such conventional optional ingredients are well-known to those skilled in the art.

A wide variety of additonal ingredients can be formulated into the present composition. These include: other conditioning agents, hair-hold polymers, additional thickening agents, aqueous carriers and suspending agents , such as xanthan gum, guar gum, hydroxyethyl cellulose, methyl cellulose, hydroxyethylcellulose, starch and starch derivatives, viscosity modifiers such as methanolamides of long chain fatty acids, cocomonoethanol amide, salts such as sodium potassium chloride and sulfate and crystalline suspending agents, and pearlescent aids such as ethylene glycol distearate; aqeoous carriers such as $C_1$ -$C_6$ alkanols; preservatives such as benzyl alcohol, methyl paraben, propyl paraben and imidazolidinyl urea; polyvinyl alcohol; ethyl alcohol; pH adjusting agents, such as citric acid, sodium citrate, succinic acid, phosphoric acid, sodium hydroxide, sodium carbonate; salts, in general, such as potassium acetate and sodium chloride; coloring agents, such as any of the FD&C or D&C dyes; hair oxidizing (bleaching) agents, such as hydrogen peroxide, perborate and persulfate salts; hair reducing agents, such as the thioglycolates; perfumes, sequestering agents, such as disodium ethylenediamine tetra-acetate, and polymer plasticizing agents, such as glycerin, disobutyl adipate, butyl stearate, and propylene glycol.

METHOD OF MAKING

The hair care compositions of the present invention can be made using conventional formulation and mixing techniques. The emulsion of the present invention is prepared by combining the silicone gum in the optional silicone fluid, adding the emulsifying agent and forming the emulsion with the addition of the aqueous carrier. The resulting emulsion is added to the other components of the hair care compositions and can be formulated into a variety of hair care products including mousses, tonics, lotions, hairsprays, and gels. Preferably the pH of the final composition should be from about 4.5 to about 9, preferably from about 4.5 to about 6.5.

Methods of making various types of hair care compositions are described more specifically in the following examples.

METHOD OF USE

The hair care compositions of the present invention are used in conventional ways to provide the conditioning and style retention benefits of the present invention. Such method of use depends upon the type of composition employed but generally involves application of an effective amount of the product to the hair, which may then be rinsed from the hair (as in the case of conditioners) or allowed to remain on the hair (as in the case of tonics, mousses, gels, lotions, and hairspray). By "effective amount" is means an amount sufficient enough to provide a conditioning and style retention benefit. In general, from about 1g to about 50g is applied to the hair. The composition is distributed throughout the hair, typically by rubbing or massaging the hair and scalp with ones' hands or by another's hands. Preferably, the composition is applied to wet or damp hair prior to drying of the hair. After such compositions are applied to the hair the hair is dried and styled in accordance with the desires of the user and in the usual ways of the user. Alternately, the composition is applied to dry hair, and the hair is then combed or styled in accordance with the desires of the user.

The following Examples further illustrate the preferred embodiments within the scope of the present invention.

EXAMPLE #1

Silicone Emulsion Premix

| Component | Weight % |
|---|---|
| Silicone blend[1] | 60.13 |
| Dimethicone Copolyol[2] | 12.03 |
| Water to | 100.00 |

[1] A 40/60 blend of high viscosity silicone gum (> 1,000,000 $mm^2/S$ (CSTK)) and silicone fluid such as SE-30 Gum and SF-18(350) Fluid available from GE Silicones.
[2] Dow Corning 190 silicone surfactant.

The emulsion is prepared entirely at ambient temperature using only low speed mixing. The dimethicone copolyol is slowly added to the highly viscous silicone blend and mixed to a uniform white opaque fluid using a conventional, three bladed, axial flow impeller at low rpm. Water is added slowly, in stepwise fashion, with continued mixing to form an emulsion.

EXAMPLE #2

Mousse Composition

| Component | Weight % |
|---|---|
| A-46 propellant[1] | 6.00 |
| Polyquatemium-4 | 1.41 |
| Silicone Emulsion from Example 1 | 0.94 |
| Lauramine Oxide | 0.188 |
| Cocamidoproply Betaine | 0.188 |
| Propylene Glycol | 0.094 |
| Preservative | Q.S. |
| Fragrance | Q.S. |
| Water | Q.S. |

[1] Available from Phillips Petroleum Company.

The aerosol mousses of the present invention are prepared by combining all ingredients except the aerosol propellant into a batch called the concentrate. This concentrate is made by combining with agitation all of the ingredients except for the preservative and the emulsion prepared as in Example I, and mixing until well dispersed. The preservative and emulsion are finally added and mixing continued until these are thoroughly dispersed.

Aerosol mousse cans are prepared by placing 135 grams of concentrate into 5 oz. aluminum epoxy lined cans, placing mousse valves on can tops, drawing a vacuum to evacuate can headspace (to remove air), and crimping the valves into place. The propellant is added by pressure filling through the valve stem.

EXAMPLE #3

Non-Aerosol Hair Tonic Spray

| Component | Weight % |
|---|---|
| Polyquaternium-4 | 1.00 |
| Carbomer 956 | 0.20 |
| Silicone Emulsion from Example 1 | 0.25 |
| Potassium Hydroxide to adjust | 6 <pH <7 |
| Preservative | Q.S. |
| Fragrance | Q.S. |
| Water | Q.S. |

A non-aerosol hair tonic spray product is prepared as follows. The polyquaternium-4 is mixed with part of the water at a ratio of about 30 to 1 until fully dissolved. The preservative is added and mixed until homogeneous. The emulsion from Example I is added and mixed until homogeneous.

The remainder of the water is put into a stainless steel mixing vessel. The Carbomer 956 is mixed into the water using, for example, a triblender or eductor mixer. Mixing is continued until the Carbomer is completely dissolved. The potassium hydroxide is added while mixing.

Then the emulsion premix is added while mixing until homogeneous. Fragrance is added and mixing is continued for an additional 10 minutes to yield the final product.

EXAMPLE #4

Conditioning Spray

| Component | Weight % |
|---|---|
| Stearyl Quaternized Protein[1] | 1.69 |
| Nonoxynol-9 | 4.00 |
| Silicone Emulsion from Example 1 | 27.00 |
| Polyquatemium-4 | 1.00 |
| Fragrance | Q.S. |
| Preservative | Q.S. |
| Water | Q.S. |

[1] Hydrotriticum Q.S.™ used as solution supplied from Croda, Inc.
[2] Available from GE Silicones.

The conditioning spray product is prepared by first dissolving the polyquatemium-4 then mixing the quaternized protein into the water. Then, the nonionic, preservative and fragrance are added. Lastly, the emulsion is blended in to give the final product.

EXAMPLE #5

Conditioner

| Component | Weight % |
|---|---|
| Cetyl Alcohol | 1.0 |
| Quaternium 18 | 0.85 |
| Stearyl Alcohol | 0.75 |
| Stearamidopropyl Dimethylamine | 0.50 |
| Ceteareth-20 | 0.35 |
| Glyceryl Monostearate | 0.25 |
| Polyquaternium-4 | 2.00 |
| Fragrance | 0.25 |
| Silicone Emulsion from Example 1 | 0.75 |
| Citric Acid | 0.13 |
| Preservative | Q.S. |
| Water | Q.S. |

Polyquaternium-4 is added to the distilled water at a temperature of 15°C to 40°C. This mixture is well-dispersed, then heated to a temperature of from 60°C to 90°C. Materials 1 through 6 are added to the batch while the temperature is maintained in this range. The mixture is stirred for approximately 10 minutes, then cooled to approximately 50°C. The remaining materials are added at this temperature. The mixture is milled under high shear for approximately 2 minutes using a conventional milling apparatus, then cooled to room temperature. The finished compositions have a pH of from about 5 to about 7.5.

EXAMPLE #6

Styling Gel

| Component | Weight % |
|---|---|
| Polyquaternium-11 | 1.00 |
| Carbomer 940 | 0.40 |
| Triethanolamine | 0.36 |
| Silicone Emulsion from Example 1 | 0.60 |
| Preservative | Q.S. |
| Fragrance | Q.S. |
| Water | Q.S. |

The gel compositions of the present invention are prepared using the method outlined in Example 3 for the hair tonic, except that the Carbomer 940 is substituted for the Carbomer 956. Only about half of the triethanolamine is added to the carbomer solution. The remaining portion is included with the other ingredients in the emulsion premix. These compositions have a pH of about 6 to 7.

EXAMPLE #7

Hair Treatment Lotion

| Component | Weight % |
|---|---|
| Hydrogenated Polyisobutylene[1] | 34.00 |
| Vitamin E Linoleate | 5.00 |
| C12-15 Alkyl Benzoate[2] | 6.00 |
| Quaternium 80[3] | 0.30 |
| Silicone Emulsion from Example 1 | 0.60 |
| PVP/VA Copolymer | 1.00 |
| Carbopol 980[4] | 0.15 |
| Sodium Hydroxide to adjust | 5.5 $<$pH $<$7 |
| Preservative | Q.S. |
| Fragrance | Q.S. |
| Water | Q.S. |

[1] Available from Amoco Chemical.
[2] Available from Finetex.
[3] Used as supplied by Goldschmidt.
[4] Available from BF Goodrich.

Components 1 through 4 plus fragrance are blended in a premix. Separately, the Carbopol is dissolved in the water as described in Example 3 and preservative is added. About half the sodium hydroxide is added and mixed to uniformity. The PVP/VA copolymer is added with mixing then the premix is added slowly, with mixing. Next, the silicone emulsion is added with continued agitation. Lastly, the remainder of the sodium hydroxide solution is added with stirring to produce a thick lotion.

EXAMPLE #8

Soft Feel Sculpting Gel

| Component | Weight % |
|---|---|
| PVP/VA Copolymer | 3.50 |
| Polyvinylpyrrolidone | 2.50 |
| Quaternium 80[1] | 0.20 |
| Isosteareth-20 | 0.50 |
| Silicone Emulsion from Example 1 | 0.60 |
| Carbomer 940 | 0.90 |
| Triethanolamine to adjust | 5.5 <pH <6.5 |
| Preservative | Q.S. |
| Fragrance | Q.S. |
| Water | Q.S. |

[1] Used as supplied by Goldschmidt.

Disperse the carbomer in about half the available water. Dissolve ingredients 1 through 4 in the remaining water; add the silicone emulsion, triethanolamine, preservative and fragrance and mix until homogeneous. Add the carbomer solution with continued agitation until homogeneous.

## Claims

**1.** A hair care composition comprising

(a) from 0.1% to 75%, by weight of the composition, of an emulsified silicone conditioning agent comprising:

(i) from 20% to 100%, by weight of the silicone conditioning agent, of a nonvolatile insoluble silicone gum having a complex viscosity at 25° C of at least 1,000,00 $mm^2/S$ (centistoke(CSTK))
(ii) from 0% to 80%, by weight of the silicone conditioning agent, of a nonvolatile insoluble silicone fluid having a viscosity at 25°C of less than 1,000,000 $mm^2/S$ (centistokes)

(b) from 0.1 % to 25%, by weight of the composition, of an emulsifying agent for said silicone conditioning agent;
(c) from 0. 1 % to 25%, by weight of the composition, of a hair setting polymer.
(d) from 0% to 25%, by weight of the composition, of an additional cationic cosmetic agent.
(e) from 24.8% to 99.7%, by weight of the composition, of an aqueous carrier;
characterized in that said hair care composition is essentially free of volatile solvents and said composition contains no more than 4%, by weight of the composition, of anionic surfactants; and said composition must contain at least 0.1% of a cationic cosmetic agent.

**2.** The composition of Claim 1 characterized in that said emulsifying agent is selected from nonionic surfactants, cationic surfactants, amphoteric surfactants, zwitterionic surfactants, and mixtures thereof; preferably selected from a nonionic surfactant and mixtures of nonionic surfactants; more preferably said emulsifying agent is silicone copolyol.

3. The composition of Claim 1 characterized in that the nonvolatile silicone conditioning agent comprises polydimethyl siloxane gum, preferably at a level of from 30% to 70%.

4. The composition of Claim 1 characterized in that the nonvolatile silicone conditioning agent comprises polydimethylsiloxane fluid having a viscosity at 25°C of 10 $mm^2$/S (centistokes) to 600,000 $mm^2$/S (centistokes), preferably a viscosity at 25°C of 100 $mm^2$/S (centistokes) to 100,000 $mm^2$/S (centistokes), preferably the polydimethylsiloxane fluid is present at a level of from 30% to 70%.

5. The composition of Claim 1 characterized in that the hair setting polymer is selected from nonionic polymers, anionic polymers, amphoteric polymers, cationic polymers and mixtures thereof; preferably selected from a cationic polymer, amphoteric polymer, and mixtures thereof; more preferably a cationic polymer.

6. The composition of Claim 5 characterized in that the hair setting polymer is present at a level of from 1% to 10%.

7. The composition of Claim 6 wherein the hair setting polymer is diallyldimonium chloride/hydroxyethylcellulose copolymer.

8. The composition of Claim 1 characterized in that the composition comprises an additional cationic cosmetic agent; preferably selected from cationic surfactants, cationic polymers, and mixtures thereof.

9. A hair care composition according to Claim 1 in the form of a lotion which additionally comprises from 0.1 % to 10.0% of a thickening agent.

10. A hair care composition according to Claim 1 in the form of a mousse.

11. A hair care composition according to Claim 1 in the form of a gel.

12. A process for making the hair care composition according to Claim 1 which comprises the steps of:

(a) combining the silicone gum in the optional silicone fluid to form a silicone conditioning agent;
(b) combining the conditioning agent with the emulsifying agent and forming the emulsion with the addition of the aqueous carrier.
(c) mixing the emulsion into a hair care composition selected from mousses, tonic, lotions, hairspray, and gels, which additionally comprise a hair setting polymer.

**Patentansprüche**

1. Haarpflegezusammensetzung, umfassend

(a) 0,1 bis 75 Gew.-% der Zusammensetzung eines emulgierten Silicon-Konditioniermittels, umfassend:

(i) 20 bis 100 Gew.-% des Silicon-Konditioniermittels eines nichtflüchtigen unlöslichen Silicongummis mit einer komplexen Viskosität bei 25°C von mindestens 1.000.000 $mm^2$/s (centistoke (CSTK)),
(ii) 0 bis 80 Gew.-% des Silicon-Konditioniermittels eines nichtflüchtigen unlöslichen Siliconfluids mit einer Viskosität bei 25°C von weniger als 1.000.000 $mm^2$/s (centistokes),

(b) 0,1 bis 25 Gew.-% der Zusammensetzung eines Emulgiermittels für das Silicon-Konditioniermittel;
(c) 0,1 bis 25 Gew.-% der Zusammensetzung eines Haar-festigenden Polymeren;
(d) 0 bis 25 Gew.-% der Zusammensetzung eines zusätzlichen kationischen kosmetischen Mittels;
(e) 24,8 bis 99,7 Gew.-% der Zusammensetzung eines wäßrigen Trägers;
dadurch gekennzeichnet, daß die Haarpflegezusammensetzung im wesentlichen frei an flüchtigen Lösungsmitteln ist und die Zusammensetzung nicht mehr als 4 Gew.-% der Zusammensetzung anionischer Tenside enthält; und daß die Zusammensetzung mindestens 0.1 % eines kationischen kosmetischen Mittels enthalten muß.

2. Zusammensetzung nach Anspruch 1, dadurch gekennzeichnet, daß das Emulgiermittel aus der nichtionische Tenside, kationische Tenside, amphotäre-Tenside, zwitterionische Tenside und Mischungen hiervon umfassenden Gruppe gewählt ist; vorzugsweise aus der nichtionische Tenside und Mischungen aus nichtionischen Tensiden umfassenden Gruppe gewählt ist; weiter vorzugsweise das Emulgiermittel Siliconcopolyol ist.

**3.** Zusammensetzung nach Anspruch 1, dadurch gekennzeichnet, daß das nichtflüchtige Silicon-Konditioniermittel Polydimethylsiloxangummi umfaßt, vorzugsweise in einem Anteil von 30 bis 70 %.

**4.** Zusammensetzung nach Anspruch 1, dadurch gekennzeichnet, daß das nichtflüchtige Silicon-Konditioniermittel Polydimethylsiloxanfluid mit einer Viskosität bei 25°C von 10 $mm^2/s$ (centistokes) bis 600.000 $mm^2/s$ (centistokes), vorzugsweise einer Viskosität bei 25°C von 100 $mm^2/s$ (centistokes) bis 100.000 $mm^2/s$ (centistokes) umfaßt, wobei vorzugsweise das Polydimethylsiloxanfluid in einem Anteil von 30 bis 70 % vorliegt.

**5.** Zusammensetzung nach Anspruch 1, dadurch gekennzeichnet, daß das Haar-festigende Polymer aus der nichtionische Polymere, anionische Polymere, amphotäre Polymere, kationische Polymere und Mischungen hiervon umfassenden Gruppe gewählt ist; vorzugsweise aus der kationisches Polymer, amphotäres Polymer und Mischungen hiervon umfassenden Gruppe gewählt ist: weiter vorzugsweise ein kationisches Polymer ist.

**6.** Zusammensetzung nach Anspruch 5, dadurch gekennzeichnet, daß das Haar-festigende Polymer in einem Anteil von 1 bis 10 % vorliegt.

**7.** Zusammensetzung nach Anspruch 6, wobei das Haar-festigende Polymer Diallyldimoniumchlorid/Hydroxyethylcellulose-Copolymer ist.

**8.** Zusammensetzung nach Anspruch 1, dadurch gekennzeichnet, daß die Zusammensetzung ein zusätzliches kationisches kosmetisches Mittel umfaßt; vorzugsweise gewählt aus der kationische Tenside, kationische Polymere und Mischungen hiervon umfassenden Gruppe.

**9.** Haarpflegezusammensetzung nach Anspruch 1 in Form einer Lotion, welche zusätzlich 0,1 bis 10,0 % eines Verdickungsmittels enthält.

**10.** Haarpflegezusammensetzung nach Anspruch 1 in Form eines Schaums.

**11.** Haarpflegezusammensetzung nach Anspruch 1 in Form eines Gels.

**12.** Verfahren zur Herstellung der Haarpflegezusammensetzung nach Anspruch 1, umfassend die Schritte:

(a) Kombinieren des Silicongummis in dem wahlweisen Siliconfluid, um ein Silicon-Konditioniermittel zu bilden;
(b) Kombinieren des Konditioniermittels mit dem Emulgiermittel und Bilden der Emulsion unter Zugabe des wäßrigen Trägers;
(c) Mischen der Emulsion zu einer Haarpflegezusammensetzung, gewählt aus der Schäume, Tonikum, Lotionen, Haarspray und Gele umfassenden Gruppe, welche zusätzlich ein Haar-festigendes Polymer umfaßt.

**Revendications**

**1.** Composition capillaire qui comprend :

(a) 0,1 à 75%, en poids de la composition, d'un agent de conditionnement émulsionné a base de silicone comprenant :

(i) 20 à 100%, en poids de l'agent de conditionnement à base de silicone, d'une gomme de silicone insoluble non volatile ayant une viscosité complexe à 25°C d'au moins 1 000 000 $mm^2/s$ (centistokes(CSTK));
(ii) 0 à 80 %, en poids de l'agent de conditionnement à base de silicone, d'un fluide à base de silicone insoluble non volatil ayant une viscosité à 25°C inférieure à 1 000 000 $mm^2/s$ (centistokes),

(b) 0,1 à 25% en poids de la composition, d'un agent émulsionnant pour ledit agent de conditionnement à base de silicone,
(c) 0,1 à 25%, en poids de la composition, d'un polymère fixant pour les cheveux,
(d) 0 à 25%, en poids de la composition, d'un agent cosmétique cationique supplémentaire,

(e) 24,8 à 99,7%, en poids de la composition, d'un véhicule aqueux;
caractérisée en ce que ladite composition capillaire est essentiellement exempte de solvants volatils et ladite composition ne contient pas plus de 4%, en poids de la composition capillaire, d'agents tensioactifs anioniques; et ladite composition doit contenir au moins 0, 1% d'un agent cosmétique cationique.

**2.** Composition selon la revendication 1, caractérisée en ce que ledit agent émulsionnant est sélectionné dans le groupe qui consiste en des agents tensioactifs non ioniques, des agents tensioactifs cationiques, des agents tensioactifs amphotères, des agents tensioactifs zwittérioniques et leurs mélanges, de préférence sélectionnés dans le groupe qui consiste en un agent tensioactif non ionique et des mélanges d'agents tensioactifs non ioniques, mieux encore ledit agent émulsionnant étant un copolyol de silicone.

**3.** Composition selon la revendication 1, caractérisée en ce que l'agent de conditionnement de silicone non volatil comprend de la gomme de polydiméthyl siloxane, de préférence à un niveau de 30 à 70%.

**4.** Composition selon la revendication 1, caractérisée en ce que l'agent de conditionnement à base de silicone non volatil comprend un fluide de polydiméthyl siloxane ayant une viscosité à 25°C de 10 $mm^2/s$ (centistokes) à 600 000 $mm^2/s$ (centistokes), de préférence une viscosité à 25°C de 100 $mm^2/s$ (centistokes) à 100 000 $mm^2/s$ (centistokes), de préférence le fluide à base de polydiméthylsiloxane étant présent à raison de 30 à 70%.

**5.** Composition selon la revendication 1, caractérisée en ce que le polymère fixant pour les cheveux est sélectionne dans le groupe consistant en des polymères non ioniques, des polymères anioniques, des polymères amphotères, des polymères cationiques et leurs mélanges, de préférence sélectionné dans le groupe consistant en un polymère cationique, un polymère amphotère et leurs mélanges, mieux encore un polymère cationique.

**6.** Composition selon la revendication 5, caractérisée en ce que le polymère fixant pour les cheveux est présent à un niveau de 1 à 10%.

**7.** Composition selon la revendication 6, dans laquelle le polylmère fixant pour les cheveux est un copolymère de chlorure de diallyldiammonium et d'hydroxyéthyl cellulose.

**8.** Composition selon la revendication 1, caractérisée en ce que la composition comprend un agent cosmétique cationique supplémentaire, de préférence sélectionné dans le groupe consistant en des agents tensioactifs cationiques, des polymères cationiques et leurs mélanges.

**9.** Composition capillaire selon la revendication 1 sous la forme d'une lotion qui comprend en outre 0,1 à 10,0% d'un agent épaississant.

**10.** Composition capillaire selon la revendication 1 sous la forme d'une mousse.

**11.** Composition capillaire selon la revendication 1 sous la forme d'un gel.

**12.** Procédé de préparation de la composition capillaire selon la revendication 1, qui comprend les étapes consistant :

(a) à combiner la gomme de silicone dans le fluide facultatif à base de silicone pour former un agent de conditionnement à base de silicone,
(b) à combiner l'agent de conditionnement avec l'agent émulsionnant et à former l'émulsion avec l'addition du véhicule aqueux, et
(c) à mélanger l'émulsion dans une composition capillaire sélectionnée dans le groupe consistant en des mousse, des toniques des lotions, des sprays pour cheveux et des gels, qui comprennent en outre un polymère fixant pour les cheveux.